# DEMANDE DE BREVET EUROPEEN

(11) **EP 1 262 168 A1**
(43) Date de publication de la demande: **04.12.2002**
(21) Numéro de dépôt: 02290537.6
(22) Date de dépôt: 05.03.2002
(51) Int. Cl.: A61K 7/48

(54) **Composition pour lutter contre le vieillissement de la peau, contenant des fibres**

(30) Priorité: 23.03.2001 FR 0103957; 23.03.2001 FR 0103958
(71) Demandeur: L'OREAL, 75008 Paris (FR)
(72) Inventeur: Chevalier, Véronique, 94440 Villecresnes (FR); Pelletier, Pascale, 92160 Anthony (FR)
(74) Mandataire: Rasson, Catherine

(57) **Abrégé**

La présente invention se rapporte à l'utilisation d'une composition topique notamment cosmétique, contenant des fibres et au moins un actif anti-vieillissement pour camoufler les imperfections de la peau et traiter les signes du vieillissement de la peau.

L'invention a aussi pour objet une composition pour application topique, contenant des fibres et au moins une vitamine choisie parmi la vitamine C, la vitamine B3, la vitamine B5, la vitamine D, la vitamine F, leurs dérivés, leurs analogues, leurs précurseurs et leurs mélanges.

## Description

La présente invention a pour objet l'utilisation d'une composition topique, notamment cosmétique, contenant des fibres et au moins un actif anti-vieillissement pour camoufler les imperfections de la peau humaine et traiter les signes du vieillissement de la peau. Elle a aussi pour objet une composition pour application topique, contenant des fibres et au moins une vitamine choisie parmi la vitamine C, la vitamine B3, la vitamine B5, la vitamine D, la vitamine F, leurs dérivés, leurs analogues, leurs précurseurs et leurs mélanges, et aux utilisations de ladite composition, notamment dans les domaines cosmétique et dermatologique.

On sait qu'au cours du processus de vieillissement, il apparaît différents signes sur la peau humaine, très caractéristiques de ce vieillissement, se traduisant notamment par une modification de la structure et de la fonction cutanées. Ce vieillissement est de nature physiologique mais il peut être également photoinduit, c'est-à-dire qu'il peut être dû à l'exposition répétée de la peau à la lumière et, par conséquent, à la formation de radicaux libres oxygénés par action de cette lumière sur les constituants de la peau.

Les principaux signes cliniques du vieillissement cutané sont notamment les suivants : apparition de ridules puis de rides profondes en augmentation avec l'âge ; désorganisation du "grain" de la peau, c'est-à-dire que le micro-relief est moins régulier et présente un caractère anisotrope ; modification du teint de la peau qui apparaît plus pâle et plus jaune, ce qui semble être dû essentiellement à une désorganisation de la microcirculation (moins d'hémoglobine au niveau du derme papillaire) ; et apparition de taches colorées en surface, ce qui est dû à une altération de la mélanogénèse.

Un autre signe clinique de vieillissement est l'aspect sec et rêche de la peau ainsi qu'une perte de fermeté et de tonicité de la peau.

Il est connu de traiter ces signes du vieillissement en utilisant des compositions cosmétiques ou dermatologiques contenant des actifs capables de lutter contre le vieillissement, tels que les agents kératolytiques comme les α-hydroxy-acides, les β-hydroxy-acides et les rétinoïdes, les vitamines et tout autre agent capable de retarder les signes du vieillissement (filtres solaires, ARL, etc...). Ces actifs agissent sur les rides et sur les autres signes du vieillissement de différente manière selon l'actif utilisé, par exemple en éliminant les cellules mortes de la peau et en accélérant le processus de renouvellement cellulaire, en protégeant la peau du soleil, en captant les radicaux libres ou en hydratant ou nourrissant la peau.

Ainsi, par exemple, l'acide ascorbique ou vitamine C stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Il est donc un excellent candidat comme actif cosmétique ou dermatologique pour lutter et/ou prévenir le vieillissement de la peau.

Par ailleurs, les vitamines B3 et B5 peuvent agir aussi pour traiter ou prévenir le vieillissement de la peau, notamment en rendant régulières la texture de la peau et/ou la taille des pores de la peau. Elles ont aussi la propriété d'enlever l'aspect huileux de la peau et de traiter les peaux grasses.

La vitamine F permet notamment de lutter contre la sécheresse de la peau.

La vitamine D est une vitamine essentielle pour la prévention et le traitement des défauts de minéralisation du cartilage (rachitisme), et de l'os (ostéomalacie), et même de certaines formes d'ostéoporose chez le sujet âgé. En application topique, la vitamine D et ses analogues permettent notamment de traiter les désordres de la peau comme par exemple le psoriasis et les signes du vieillissement.

Toutefois, la plupart des actifs antivieillissement présentent l'inconvénient de n'être efficaces pour le traitement des signes du vieillissement de la peau qu'après un certain temps d'application. Or, on cherche de plus en plus à obtenir un effet immédiat des actifs utilisés, conduisant rapidement à un lissage des rides et ridules et à la disparition des marques de fatigue, à l'estompage des signes apparents du vieillissement, afin d'obtenir une peau lisse et qui respire la bonne santé.

La demanderesse a trouvé de manière surprenante que l'association de fibres et d'actifs anti-vieillissement permettait d'obtenir un effet à la fois immédiat et à long terme sur les signes visibles du vieillissement (ridules, rides, taches, teint terne) et de toute autre imperfection de la matière kératinique traitée et notamment de la peau, tout en gardant une apparence naturelle à la peau. En outre, les fibres permettent d'apporter de bonnes propriétés cosmétiques : douceur et confort lors de l'application sur la peau, et facilité d'application.

La présente invention a donc pour objet l'utilisation cosmétique d'une composition cosmétique contenant des fibres et au moins un actif anti-vieillissement, pour camoufler les imperfections de la peau et traiter les signes du vieillissement de la peau.

L'invention a aussi pour objet l'utilisation de fibres et d'au moins un actif anti-vieillissement, pour la préparation d'une composition pour application topique destinée au traitement du vieillissement de la peau tout en camouflant les signes du vieillissement.

On entend ici par « application topique » une application externe sur les matières kératiniques, et par « matières kératiniques », on entend notamment la peau, le cuir chevelu, les cils, les sourcils, les ongles et les muqueuses. La matière kératinique est de préférence la peau.

L'invention a aussi pour objet un procédé de traitement cosmétique des signes du vieillissement des matières kératiniques et notamment de la peau, caractérisé en ce qu'il consiste à appliquer sur les matières kératiniques, une composition cosmétique contenant des fibres et au moins un actif anti-vieillissement.

La composition utilisée selon l'invention étant destinée à une application topique, elle comprend un milieu physiologiquement acceptable, c'est-à-dire compatible avec toutes les matières kératiniques telles que la peau y compris le cuir chevelu, les ongles, les muqueuses, les yeux et les cheveux ou toute autre zone cutanée du corps. Elle peut constituer notamment une composition cosmétique ou dermatologique.

On entend ici par « actif anti-vieillissement », tout actif susceptible de traiter ou de prévenir tout signe de vieillissement de la peau y compris du cuir chevelu, des cheveux ou des muqueuses (lèvres), notamment tout agent susceptible de traiter et/ou de prévenir les rides et ridules de la peau, de donner un teint clair et de lisser la peau.

### Fibres

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Leur forme ou morphologie peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres peuvent avoir une longueur (L) allant de 1 µm (0,001 mm) à 10 mm, de préférence de 0,1 µm à 5 mm et mieux de 0,1 mm à 1,5 mm. Leur section peut être comprise dans un cercle de diamètre (D) allant de 1 nm (0,001 µm) à 100 µm, de préférence allant de 1 nm (0,001 µm) à 50 µm et mieux de 5 µm à 40 µm.

De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles et notamment des fibres de soie, de coton, de laine, de lin, de cellulose extraites notamment du bois, des légumes ou des algues, de polyamide (Nylon®), de cellulose modifiée (rayonne, viscose, acétate notamment d'acétate de rayonne), de poly-p-phénylène téréphtalamide notamment de Kevlar®, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester.

Comme fibres de polyuréthane, on peut citer les fibres en polymère poly(uréthane-urée) segmenté, appartenant à la classe des élastanes, et notamment celles commercialisées sous le nom Lycra® par la société Du PONT.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

On peut aussi utiliser des mélanges des fibres citées ci-dessus.

Par ailleurs, les fibres peuvent être traitées ou non en surface, enrobées ou non. Il peut s'agir notamment de fibres enrobées et/ou fonctionnalisées, « fonctionnalisées » signifiant que les fibres sont traitées en surface afin d'en modifier les propriétés.

Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Les fibres peuvent être également fonctionnalisées, c'est-à-dire être modifiées de manière à avoir une fonction spécifique. Cette fonctionnalisation des fibres peut être réalisée aussi bien sur les fibres que dans les fibres, et ce par toute méthode permettant d'accrocher un composé aux fibres ou de le piéger dans les cavités formées par la géométrie des fibres. Comme méthodes, on peut citer par exemple l'enduction des fibres par un actif ; la fixation de particules renfermant un actif, telles que nanocapsules ou nanosphères, sur les fibres ; l'adsorption dans les fibres ; la fixation par réaction chimique. On peut ainsi utiliser des fibres ayant des fonctionnalités particulières, par exemple des fibres anti-UV par modification avec filtres solaires chimiques ou physiques ; des fibres bactéricides ou antiseptiques par modification avec des conservateurs ou d'anti-bactériens ; des fibres colorées par modification avec des molécules colorantes ; des fibres kératolytiques ou desquamantes par modification avec des agents kératolytiques ou desquamants ; des fibres hydratantes par modification avec des agents hydratants ou des polymères rétenteurs d'eau ; les fibres parfumées par modification avec un parfum ; des fibres analgésiques ou apaisantes par modification avec un antiinflammatoire ou un agent apaisant ; des fibres anti-transpirantes par modification avec un anti-transpirant.

Selon leurs propriétés, les fibres utilisées selon la présente invention peuvent être introduites dans un milieu aqueux, un milieu huileux ou dans une poudre.

Les fibres utilisables selon l'invention sont de préférence choisies parmi les fibres de polyamide, les fibres de poly-p-phénylène téréphtalamide, les fibres de coton et leurs mélanges. Leur longueur peut aller de 0,1 à 10 mm, de préférence de 0,1 à 1 mm, leur diamètre moyen peut aller de 5 à 50 µm et le facteur de forme va de préférence de 5 à 150.

En particulier, on peut utiliser les fibres de polyamide commercialisées par les Etablissements P. Bonte sous le nom Polyamide 0,9 dtex 0,3 mm, ayant un diamètre moyen de 15 à 20 µm, un titre d'environ 0,9 dtex (0,81 denier) et une longueur allant de 0,3 mm à 1,5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre.

On peut aussi utiliser des fibres de coton par exemple celles ayant un diamètre moyen de 20 µm, une longueur de 0,3 mm, et un facteur de forme de 15, telles que celles commercialisées par l'Institut Textile de France, la société Filature de Lomme, par la société Textiles des Dunes ou par la société Velifil.

Les fibres peuvent être présentes dans la composition selon l'invention en une quantité allant par exemple de 0,01 à 50 % en poids, de préférence de 1 à 20 % en poids et mieux de 5 à 10 % en poids de matière active par rapport au poids total de la composition.

### Actifs anti-vieillissement

Comme indiqué plus haut, l'actif anti-vieillissement peut être tout actif susceptible de traiter ou de prévenir tout signe de vieillissement de la peau.

Les actifs peuvent être choisis par exemple parmi les agents hydratants, les agents anti-radicaux libres, les agents kératolytiques, les vitamines, les agents anti-élastase et anti-collagénase, les protides, les dérivés d'acides gras, les stéroïdes, les oligo-éléments, les agents blanchissants, les extraits d'algues et de planctons, les filtres solaires, les enzymes et co-enzymes, les flavonoïdes, les céramides, et leurs mélanges.
I. Comme agents hydratants, on peut citer en particulier le lactate de sodium ; les polyols, et en particulier la glycérine, le sorbitol, les polyéthylène glycols ; le mannitol ; les acides aminés ; l'acide hyaluronique ; la lanoline ; l'urée et les mélanges contenant de l'urée, tels que le NMF (« Natural Moisturising Factor ») ; la vaseline ; et leurs mélanges.
II. Comme agents anti-radicaux libres, on peut citer notamment les dérivés de l'acide phosphonique tels que l'acide éthylènediamine tétra(méthylène phosphonique), l'acide hexaméthylènediamine tétra(méthylène phosphonique), l'acide diéthylènetriamine penta(méthylènephosphonique) et leurs sels et en particulier leurs sels de sodium ; l'acide éthylènediamine tétracétique et ses sels tels que le sel de sodium ; la guanosine ; la superoxydismutase ; le tocophérol (vitamine E) et ses dérivés (acétate) ; l'éthoxyquine ; la lactoferrine ; la lactoperoxydase, et les dérivés nitroxydes; les superoxyde dismutases ; le glutathion peroxydase ; les extraits végétaux à activité antiradicalaire tels que l'extrait aqueux de germe de blé commercialisé par la société Silab sous la référence Detoxiline ; et leurs mélanges.
III. Comme agents kératolytiques, on peut citer par exemple les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5 salicylique ; les α-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199 636, EP-A-325 540, EP-A-402 072 ; et leurs mélanges.
IV. Comme vitamines, outre les vitamines A, E et C indiquées ci-dessus, on peut citer en particulier la vitamine B3 (ou vitamine PP ou niacinamide), la vitamine B5 (ou panthénol), la vitamine D, la vitamine F, les dérivés, précurseurs et analogues de ces vitamines ainsi que ceux des vitamines A, E et C, comme les lycopènes ou les carotènes précurseurs de la vitamine A, et leurs mélanges.
   - La vitamine B3, encore appelée vitamine PP est un composé de formule : 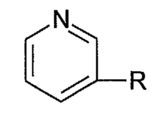
   dans laquelle R peut être -CONH₂ (niacinamide), -COOH (acide nicotinique ou niacine), -CH₂OH (alcool nicotinyle), -CO-NH-CH₂-COOH (acide nicotinurique) ou - CO-NH-OH (acide niconityl hydroxamique).
   Comme dérivés de la vitamine B3, on peut citer par exemple les esters de l'acide nicotinique, tels que le nicotinate de tocophérol ; les amides dérivés de la niacinamide par substitution des groupes hydrogène de - CONH₂; les produits de réaction avec les acides carboxyliques et les acides aminés ; les esters d'alcool nicotinyle et d'acides carboxyliques tels que l'acide acétique, l'acide salicylique, l'acide glycolique, l'acide palmitique. On peut citer aussi les dérivés suivants : 2-chloronicotinamide, 6-méthylnicotinamide, 6-aminonicotinamide, N-méthyl-nicotinamide, N,N-diméthylnicotinamide, N-(hydroxyméthyl)-nicotinamide, imide d'acide quinolinique, nicotinanilide, N-benzylnicotinamide, N-éthylnicotinamide, nifenazone, nicotinaldéhyde, acide isonicotinique, acide méthylisonicotinique, thionicotinamide, nialamide, acide 2-mercaptonicotinique, nicomol et niaprazine.
   Comme autres dérivés de la vitamine B3, on peut citer également ses sels inorganiques tels que les chlorures, bromures, iodures, carbonates, et ses sels organiques tels que les sels obtenus par réaction avec des acides carboxyliques tels que acétate, salicylate, glycolate, lactate, malate, citrate, mandélate, tartrate, etc).
   - Comme vitamine B5, on peut utiliser le panthénol ou alcool panthénylique ou 2,4-dihydroxy-N-(3-hydroxypropyl)-3,3diméthylbutanamide, sous ses différentes formes : D-panthénol, DL-panthénol, et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale.
   - Comme vitamine D, on peut citer la 1α,25-dihydroxyvitamine D3 et ses analogues, ainsi que les analogues de vitamine D, tels que ceux décrits dans le document WO-A-00/26167, comme par exemple :
   - 3-hydroxyméthyl-5-{2-[3-(5-hydroxy-5- ou 6-méthyl-hexyl)-phényl]-vinyl}-phenol,
   - 3-[3-(5-hydroxy-1,5-diméthyl-hexyl)-phenoxyméthyl]-5-hydroxyméthyl-phenol,
   - 6-[3-(3,4-bis-hydroxyméthyl-benzyloxy)-phényl]-2-méthyl-hepta-3,5-dien-2-ol,
   - 6-[3-(3,4-bis-hydroxyméthyl-benzyloxy)-phényl]-2-méthyl-hexan-2-ol,
   - 6-[3-(3,4-bis-hydroxyméthyl-phenoxyméthyl)-phényl]-2-méthyl-heptan-2-ol,
   - 7-[3-(3,4-bis-hydroxyméthyl-phénoxyméthyl)-phényl]-3-éthyl-octan-3-ol,
   - 5-{2-[4-(5-hydroxy-5-méthyl-hexyl)-phényl]-vinyl ou -éthyl}-benzène-1,3-diol,
   - 5-{2-[3- ou 4-(6-hydroxy-6-méthyl-heptyl)-phényl]vinyl}-benzene-1,3-diol,
   - 5-{2-[3- ou 4-(6-hydroxy-6-méthyl-heptyl)-phényl]-éthyl}-benzène-1,3-diol,
   - 2-hydroxyméthyl-4-{2-[3- ou 4-(5-hydroxy-5-méthyl-hexyl)-phényl]-vinyl}-phenol,
   - 2-hydroxyméthyl-4-{2-[3 ou 4-(6-hydroxy-6-méthyl-heptyl)-phényl]-vinyl}-phénol,
   - 2-hydroxyméthyl-4-{2-[3- ou 4-(5-hydroxy-5-méthyl-heptyl)-phényl]-éthyl}-phénol,
   - 2-hydroxyméthyl-4-{2-[3- ou 4-(6-hydroxy-6-méthyl-heptyl)-phényl]-éthyl}-phénol,
   - 2-hydroxyméthyl-5-{2-[4-(5-hydroxy-5-méthyl-hexyl)-phényl]-vinyl}-phénol,
   - 6-[3-(3,4-bis-hydroxyméthyl-benzyloxy)-phényl]-2-méthyl-heptan-2-ol,
   - 4-[3-(5-hydroxy-1,5-diméthyl-hexyl)-phenoxyméthyl]-2-hydroxyméthyl-phenol,
   - 6-{3- ou 4-[2-(3,4-bis-hydroxyméthyl-phényl)-vinyl]-phényl}-2-méthyl-hexan-2-ol,
   - 7-{4-[2-(3,4-bis-hydroxyméthyl-phényl)-vinyl]-phényl}-2-méthyl-heptan-2-ol,
   - 5-{2-[3-(6-hydroxy-6-méthyl-heptyl)-phényl]-1-méthyl-vinyl}-benzène-1,3-diol,
   - 5-{2-[3-(5-hydroxy-5-méthyl-hexyl)-phényl]-vinyl}-benzène-1,3-diol,
   - 5-[3-(6-hydroxy-6-méthyl-heptyl)-phénoxyméthyl]-benzène-1,3-diol,
   - 5-{2-[3-(7-hydroxy-7-méthyl-oct-1-enyl)-phényl]-vinyl}-benzene-1,3-diol,
   - 5-{2-[3-(7-hydroxy-7-méthyl-octyl)-phényl]-vinyl}-benzène-1,3-diol,
   - 4-{2-[3-(6-hydroxy-6-méthyl-heptyl)-phényl]-vinyl}-benzène-1,2-diol,
   - 3-{2-[3-(6-hydroxy-6-méthyl-heptyl)-phényl]-vinyl}-phénol,
   - 6-{3-[2-(3,5-bis-hydroxyméthyl-phényl)-vinyl]-phényl}-2-méthyl-hexan-2-ol,
   - 3-{2-[3-(7-hydroxy-7-méthyl-octyl)-phényl]-vinyl}-phénol,
   - 7-{3-[2-(3,5-bis-hydroxyméthyl-phényl)-vinyl]-phényl}-2-méthyl-heptan-2-ol,
   - 7-{3-[2-(3,4-bis-hydroxyméthyl-phényl)-vinyl]-phényl}-2-méthyl-heptan-2-ol,
   - 7-{3-[2-(4-hydroxyméthyl-phényl)-vinyl]-phényl}-2-méthyl-heptan-2-ol,
   - 4-{2-[3-(7-hydroxy-7-méthyl-oct-1-enyl)-phényl]-vinyl}-benzene-1,2-diol,
   - 7-[3-(3,4-bis-hydroxyméthyl-phényléthynyl)-phényl]-2-méthyl-heptan-2-ol,
   - 5-{2-[3-(6-hydroxy-6-méthyl-hept-1-enyl)-phényl]-vinyl}-benzene-1,3-diol,
   - 5-{2-[3-(7-éthyl-7-hydroxy-non-1-enyl)-phényl]-vinyl}-benzène-1,3-diol,
   - 5-{2-[3-(7-hydroxy-1-méthoxy-1,7-diméthyl-octyl)-phényl]-vinyl}-benzène-1,3-diol,
   - 5-{2-[3-(6-hydroxy-1-méthoxy-1,6-diméthyl-heptyl)-phényl]-vinyl}-benzène-1,3-diol,
   - 5-{2-[3-(5-hydroxy-pentyl)-phényl]-vinyl}-benzène-1,3-diol,
   - 5-{2-[3-(5-hydroxy-6-méthyl-heptyl)-phényl]-vinyl}-benzène-1,3-diol,
   - 5-{2-[3-(6-hydroxy-7-méthyl-octyl)-phényl]-vinyl}-benzène-1,3-diol,
   - 5-{2-[3-(5-hydroxy-6-méthyl-hept-1-enyl)-phényl]-vinyl}-benzène-1,3-diol,
   - 5-{2-[3-(6-hydroxy-7-méthyl-oct-1-enyl)-phényl]-vinyl}-benzène-1,3-diol,
   - 5-{2-[3-(1,6-dihydroxy-1,6-diméthyl-heptyl)-phényl]-vinyl}-benzène-1,3-diol,
   - 5-{2-[3-(6-hydroxy-1,6-diméthyl-hept-1-enyl)-phényl]-vinyl}-benzène-1,3-diol.
   - La vitamine F est un mélange d'acides gras essentiels, c'est-à-dire d'acides insaturés possédant au moins une double liaison, tels que l'acide linoléique ou acide 9,12-octadécadiènoïque et ses stéréoisomères, l'acide linolénique sous forme α (acide 9,12,15-octadécatriènoïque) ou γ (acide 6,9,12- octadécatriènoïque) et leurs stéréoisomères, l'acide arachidonique ou acide 5,8,11,14-eicosatétraènoïque et ses stéréosiomères.

   On peut utiliser dans la composition de la présente invention, la vitamine F ou ses analogues tels que les mélanges d'acides insaturés possédant au moins une double liaison et notamment les mélanges d'acide linoléique, d'acide linolénique et d'acide arachidonique, ou les composés en contenant et notamment les huiles d'origine végétale en contenant, telles que par exemple l'huile de jojoba.

V. Comme agents anti-élastase, on peut citer notamment les dérivés peptidiques, et notamment les peptides de graines de légumineuses tels que ceux commercialisés par les Laboratoires Sériobiologiques de Nancy sous la référence Parelastyl ; les dérivés de N-acylamino-amides décrits dans la demande FR-A-2,180,033, comme par exemple le {2-[acétyl-(3-trifluorométhyl-phényl)-amino]-3-méthyl-butyrylamino} acétate d'éthyle et l'acide {2-[acétyl-(3-trifluorométhylphényl)-amino]-3-méthyl-butyryl-amino} acétique, et leurs mélanges. Comme agents anti-collagénase, on peut citer les inhibiteurs de métalloprotéase, tels que l'acide éthylènediamine (EDTA), la cystéine, et leurs mélanges.
VI. Comme protides, on peut citer par exemple les protéines (de blé ou de soja), leurs hydrolysats, comme ceux commercialisés par la société Silab sous le référence Tensine, et leurs mélanges.
VII. Comme dérivés d'acide gras, on peut citer notamment les phospholipides polyinsaturés dont les phospholipides d'acide gras essentiels de poulpe, et leurs mélanges.
VIII. Comme stéroïdes, on peut citer par exemple la DHEA ou déhydroépiandrostérone, ses précurseurs biologiques, ses métabolites, et leurs mélanges. Par "précurseurs biologiques" de la DHEA, on entend notamment la Δ5-prégnénolone, la 17α-hydroxy prégnénolone et le sulfate de 17α-hydroxy prégnénolone. Par dérivés de la DHEA, on entend aussi bien ses dérivés métaboliques que ses dérivés chimiques. Comme dérivés métaboliques, on peut citer notamment le Δ5-androstène-3,17-diol et notamment le 5-androstène 3β, 17β-diol, la Δ4-androstène-3,17-dione, la 7 hydroxy DHEA (7α-hydroxy DHEA ou 7β-hydroxy-DHEA) et la 7-céto-DHEA qui est elle-même un métabolite de la 7β-hydroxy DHEA.
IX. Comme oligo-éléments, on peut par exemple le cuivre, le zinc, le sélénium, le fer, le magnésium, le manganèse, et leurs mélanges.
X. Comme agents blanchissants, on peut utiliser tout composé permettant de traiter ou de prévenir les taches de vieillissement, c'est-à-dire tout composé dépigmentant qui agit directement sur la vitalité des mélanocytes épidermiques où se déroule la mélanogénèse et/ou qui interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation. On peut citer par exemple comme actif blanchissant, l'acide kojique et ses dérivés, l'hydroquinone et ses dérivés tels que l'arbutine et ses esters ; l'acide ellagique et ses dérivés ; les extraits de plantes, et notamment de réglisse, de mûrier ou de scutellaire ; le glutathion et ses précurseurs; la cystéine et ses précurseurs ; les composés dérivés d'aminophénol décrits dans le document WO-A-99/10318, comme notamment le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylaminocarbonyl-4-aminophénol ; et les mélanges de ces composés.
XI. Comme extraits d'algues, on peut citer les extraits d'algues rouges ou brunes, et par exemple l'extrait d'algues brunes de la famille des Laminaires, comme les extraits de l'espèce *Laminaria digitata,* et plus particulièrement celui vendu par la société CODIF sous la dénomination PHYCOSACCHARIDES, qui est une solution concentrée d'un oligosaccharide obtenu par dépolymérisation enzymatique contrôlée de polysaccharides membranaires d'une algue brune. Il comprend l'enchaînement de deux acides uriques : acide mannuronique et l'acide guluronique.
XII. Comme extraits de planctons, on peut citer le plancton en dispersion aqueuse (nom CTFA : Vitreoscilla Ferment) commercialisé sous la dénomination MEXORYL SAH par la société Chimex.
XIII. Comme filtres solaires, on peut utiliser dans la composition selon l'invention tous les filtres chimiques UVA et UVB ou filtres physiques habituellement utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX ;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(tert.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-1 H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolylbenzoxazole ;
   - le 2-[1-(3-trimethylsilanyl-propyl)-1H-benzimidazol-2-yl]-benzothiazole, qui sont décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) Les silicones benzotriazoles, qui sont décrits notamment dans la demande de brevet EP-A- 0392 883, en particulier le silicone benzotriazole de formule : 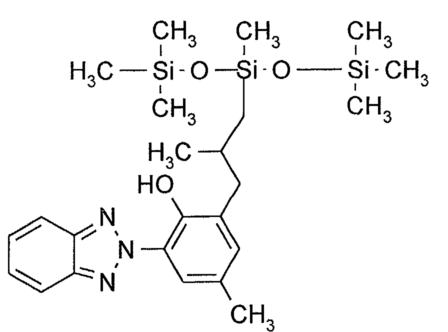
(10) leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence des nano-pigments.

XIV. Comme enzymes, on peut utiliser toute enzyme d'origine animale, microbiologique (bactérienne, fongique ou virale) ou synthétique (obtenue par synthèse chimique ou biotechnologique), sous forme cristalline pure ou sous une forme diluée dans un diluant inerte. On peut citer par exemple parmi les lipases, les protéases, les phospholipases, les cellulases, les peroxydases notamment les lactoperoxydases, les catalases, les superoxyde dimutases, ou parmi des extraits végétaux contenant les enzymes précitées, et leurs mélanges. Elles peuvent être choisies par exemple parmi celle vendue sous la dénomination commerciale « Subtilisine SP 554 » par la société Novo Nordisk et celle vendue sous la dénomination commerciale « LYSOVEG LS » par la société Laboratoires Sérobiologiques de Nancy.

Comme co-enzymes, on peut utiliser notamment l'ubiquinone ou coenzyme Q10 qui appartient à la famille des benzoquinones à chaine alkylénée, le coenzyme R qui est la biotine (ou vitamine H), et leurs mélanges.

XV. Comme flavonoïdes, on peut citer par exemple les isoflavonoïdes qui constituent une sous-classe des flavonoïdes, formés d'un squelette 3-phényl chromane qui peut comporter des substituants variés et différents niveaux d'oxydation. Le terme « isoflavonoïde » regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les α-méthyldeoxybenzoines, les 2-arylbenzofuranes, et leurs mélanges. A cet égard on se reportera avantageusement pour une revue complète sur les isoflavonoïdes, leurs méthodes d'analyse et leurs sources, au chapitre 5 "Isoflavonoïds" écrit par P.M. Dewick, dans The Flavonoïds, Harbone éditeur, pp. 125-157 (1988).

Les isoflavonoïdes peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique.

On préfère utiliser les isoflavonoïdes d'origine naturelle. Parmi ceux-ci, on peut citer : la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.

XVI. Comme céramides, on peut utiliser tout type de céramide, d'origine naturelle ou synthétique, par exemple de type II, de type III, de type IV, de type V ou de type VI, et leurs mélanges.

On peut citer par exemple comme céramides, la N-oléoyl-dihydrosphingosine, la N-stéaroyl phytosphingosine, le N-α-hydroxybéhénoyl-dihydrosphingosine, la N-α-hydroxypalmitoyl-dihydrosphingosine, la N-linoléoyidihydrosphingosine, la N-palmitoyidihydrosphingosine, la N-stéaroyidihydrosphingosine, la N-béhénoyidihydrosphingosine, et leurs mélanges.

On peut citer aussi le produit constitué d'un mélange de glycocéramides, vendu sous la dénomination commerciale GLYCOCER par la société WAITAKI INTERNATIONAL BIOSCIENCES ; les composés décrits dans les documents EP-A-0227994 et WO-A-94/07844, comme par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique ; le N-docosanoyl N-méthyl-D-glucamine décrit dans la demande de brevet WO-A-92/05764.

On peut aussi utiliser les mélanges de ces céramides.

La composition de l'invention peut comprendre un ou plusieurs actifs antivieillissement, de la même catégorie ou de différentes catégories. La quantité d'actif(s) dans la composition dépend de l'actif utilisé et de la finalité de la composition. Le ou les actifs doivent être en quantité efficace, c'est-à-dire en une quantité suffisante pour atteindre le but recherché qui est l'amélioration de l'apparence de la peau et le traitement de signes du vieillissement de la peau. La quantité d'actif(s) dans la composition peut aller par exemple de 0,0001 à 30 % en poids, de préférence de 0,01 à 20 % en poids, mieux de 0,1 à 15 % en poids et encore mieux de 0,5 à 10 % en poids de matière active par rapport au poids total de la composition.

Selon un mode particulier de réalisation, l'actif anti-vieillissement est une vitamine choisie parmi les vitamines C, B3, B5, D, F et leurs dérivés.

Aussi, la présente invention a encore pour objet une composition pour application topique, contenant des fibres et au moins une vitamine choisie parmi la vitamine C, la vitamine B3, la vitamine B5, la vitamine D, la vitamine F, leurs dérivés, leurs précurseurs et leurs mélanges. Cette composition peut constituer une composition cosmétique ou dermatologique, de préférence une composition cosmétique pouvant constituer en particulier une composition anti-vieillissement.

Ainsi, la composition de l'invention contenant ces vitamines peut être utilisée notamment pour lutter et/ou prévenir le vieillissement de la peau, notamment en diminuant les ridules et les rides, en atténuant, voire en éliminant les taches apparaissant au cours du temps, en protégeant la peau des rayonnements U.V., en tonifiant la peau, en régénérant les tissus cutanés, en donnant un éclat au teint, en rendant réguliers la texture de la peau et/ou la taille des pores de la peau.

Ainsi, l'invention a encore pour objet l'utilisation cosmétique de la composition telle que définie ci-dessus pour un traitement cosmétique de la peau en vue de la tonifier, de la régénérer, de lisser les ridules de la peau, d'éclaircir le teint, d'atténuer les taches pigmentaires de la peau, de protéger la peau des rayonnements U.V., et/ou de rendre réguliers la texture de la peau et/ou la taille des pores de la peau.

L'invention a encore pour objet un procédé de traitement cosmétique des signes du vieillissement d'une matière kératinique, caractérisé en ce qu'il consiste à appliquer sur la matière kératinique, une composition telle que définie ci-dessus.

Le milieu physiologiquement acceptable des compositions à application topique, selon l'invention peut plus particulièrement comprendre de l'eau et éventuellement un solvant organique physiologiquement acceptable choisi par exemple parmi les alcools inférieurs comportant de 1 à 8 atomes de carbone et de préférence 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol.

Ce milieu peut être aussi un milieu anhydre, notamment un milieu huileux contenant des huiles et/ou matières grasses autres que les huiles, comme décrit ci-dessous.

Selon un mode préféré de réalisation de l'invention, le milieu de la composition comprend de l'eau. Ce milieu aqueux a de préférence un pH compatible avec la peau, allant de préférence de 3 à 8 et mieux de 4,5 à 7.

Quand la composition comporte un milieu aqueux ou hydroalcoolique, il est possible d'ajouter une phase grasse (ou huileuse) à ce milieu, afin que les compositions de l'invention soient plus douces et plus nourrissantes.

La phase huileuse contient habituellement au moins une huile. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle, le néopentanoate d'isostearyle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les compositions selon l'invention peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple (E/H/E ou H/E/H), de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Selon un mode particulier de réalisation de l'invention, la composition selon l'invention est une émulsion. La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de Polyglyceryl-4 isostearate/Cetyl dimethicone copolyol/Hexyl laurate vendu sous la dénomination Abil WE 09^{R} par la société Goldschmidt, le mélange de cyclomethicone et de dimethicone copolyol, vendu sous les dénominations DC 5225 C et DC 3225 C par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose, et leurs mélanges comme par exemple le mélange de stéarate de glycéryle et de stearate de PEG-100, commercialisé sous la dénomination Arlacel 165 par la société Uniqema.

On peut aussi préparer des émulsions sans tensioactifs en utilisant des composés appropriés, par exemple les polymères ayant des propriétés émulsionnantes tels que le carbopol 1342 et les PEMULEN, et les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate / 1,4-cyclohexane-diméthanol; nom CTFA: Diglycol/CHDM/lsophtalates/SIP Copolymer) vendus sous les dénominations "Eastman AQ polymer" (AQ35S, AQ38S, AQ55S, AQ48 Ultra) par la société Eastman Chemical.

De façon connue, les compositions cosmétiques ou dermatologiques de l'invention peuvent contenir également des adjuvants habituels dans le domaine cosmétique ou dermatologique, tels que les gélifiants hydrophiles ou lipophiles, les actifs hydrophiles ou lipophiles autres que ceux cités ci-dessus, les conservateurs, les antioxydants, les solvants, les parfums, les charges, les bactéricides, les absorbeurs d'odeur, les matières colorantes, les sels, les polymères (par exemple Acrylates/Dimethicone Copolymer commercialisé sous la dénomination KP-561 par Shin-Etsu, comme dispersant). Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme gélifiants, on peut citer par exemple les polymères carboxyvinyliques tels que les carbopols (carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic; les polymères et copolymères d'acide 2-acrylamido 2-méthylpropane sulfonique), éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; les argiles (bentone, laponite, saponite, etc..) et leurs mélanges. Ces charges peuvent être présentes dans des quantités allant de 0 à 20 % en poids et de préférence de 1 à 10 % en poids par rapport au poids total de la composition.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 : Emulsion triple

| 1. Emulsion primaire : | |
|---|---|
| *Phase A :* | |
| - Abil WE 09 | 2,5 % |
| - Huile de Parléam | 17,5 % |
| - Polydiméthylsiloxane | 4 % |

| *Phase B :* | |
|---|---|
| - Glycérine | 39 % |
| - Séquestrant | 0,1 % |
| - conservateur | 0,8 % |
| - Eau déminéralisée | 36,1 % |

| 2. Emulsion triple : | |
|---|---|
| *Phase A :* | |
| - Emulsion primaire | 20 % |
| - Huile de Parléam | 5 % |
| - Rétinol | 0,1 % |

| *Phase B :* | |
|---|---|
| - Hostacerin AMPS | 0,5 % |
| - Copolymère acrylate/C₁₀-C₃₀-alkylacrylate (Pemulen TR1) | 0,3 % |
| - Conservateurs | 1 % |
| - Eau déminéralisée | 40 % |

| *Phase C :* | |
|---|---|
| - triéthanolamine | 0,3 % |
| - Eau déminéralisée | 2 % |

| *Phase D :* | |
|---|---|
| - Hostacerin AMPS | 1,5 % |
| - Eau déminéralisée | qsp 100 % |

| Phase E | |
|---|---|
| Fibres de polyamide | |
| (Polyamide 0.9dtex, 0,3mm - société Paul Bonte) | 5 % |

L'émulsion triple est préparée de la manière suivante :
1. On prépare l'émulsion primaire en mélangeant les constituants de la phase A à température ambiante, en mélangeant par ailleurs les constituants de la phase B à température ambiante et en versant lentement la phase B dans la phase A sous agitation rapide.
2. Pour préparer l'émulsion triple, on prépare les différentes phases, puis on verse lentement la phase A dans la phase B sous agitation rapide. On y ajoute la phase C, puis la phase D et enfin la phase E à environ 40°C. On agite jusqu'à homogénéisation complète.

On obtient une crème hydratante apte à améliorer l'éclat du teint et à estomper les signes du vieillissement.

### Exemple 2 : on peut préparer une composition analogue à celle de l'exemple 1 en remplaçant le rétinol par l'acide glycolique.

### Exemple 3 : Emulsion H/E

| *Phase A :* | |
|---|---|
| Hydrogenated polyisobutene | 5,5 % |
| Isostearyl neopentanoate | 3,5 % |
| PEG-20 stearate | 1 % |
| Glyceryl stearate et PEG-100 stearate (Arlacel 165) | 2 % |
| Cetyl alcohol | 0,5 % |
| Stearyl alcohol | 0,5 % |
| Stearic acid | 1 % |

| *Phase A':* | |
|---|---|
| Cyclomethicone | 11 % |
| Fibres de coton | 3 % |

| *Phase B :* | |
|---|---|
| Conservateurs | qs |
| Triéthanolamine | 0,03 % |
| Eau | qsp 100 % |

| *Phase C :* | |
|---|---|
| SEPIGEL 305 | 1 % |
| Parelastyl | 5 % |

Mode opératoire : On chauffe la phase A sous agitation jusqu'à homogénéité. Après refroidissement on ajoute la phase A'. On chauffe la phase B sous agitation, puis on verse B dans A toujours sous agitation. Après refroidissement à 50°C, on incorpore à l'émulsion la phase C.

On obtient une crème apte à estomper et traiter les rides et ridules.

### Exemple 4 : Emulsion fluide

| *Phase A* | |
|---|---|
| - N-éthyloxycarbonyl-4-aminophénol | 0,5 % |
| - Mélange d'alcools cétylique et stéarylique | |
| oxyéthylénés (20 OE) | 2 % |
| - Huile de ricin hydrogénée oxyéthylénée (60 OE) | 2,5 % |

| Phase A' | |
|---|---|
| - Huile d'abricot | 5 % |
| - Filtres UV | 3,9 % |
| - Huile d'abricot | 5 % |
| - Cyclohexasiloxane | 10 % |
| - Sesquistéarate de méthylglucose polyéthoxylé (20 OE) | 2 % |

| *Phase B* | |
|---|---|
| - Conservateurs | 0,65 % |
| - Triéthanolamine | 0,5 % |
| - EDTA disodique | 0,05 % |
| - Glycérine | 5 % |
| - Eau déminéralisée | qsp 100 % |

| *Phase C* | |
|---|---|
| - Gélifiants | 4,25 % |

| *Phase D* | |
|---|---|
| - Fibres de polyamide | |
| (Polyamide 0.9dtex, 0,3mm - société Paul Bonte) | 5 % |

La composition est préparée de la manière suivante : la phase A est chauffée à environ 80°C jusqu'à dissolution complète, puis introduite dans la phase B préalablement portée à la même température. La phase A' est chauffée à environ 80°C, puis introduite dans le mélange préalablement obtenu, pour former une émulsion H/E à laquelle les phases C et D sont ensuite ajoutées.

L'émulsion obtenue est fluide et elle permet d'estomper les taches pigmentaires sur le visage, le décolleté et les mains.

### Exemple 5 : Emulsion E/H

| *Phase A* | |
|---|---|
| Mélange de diméthicone copolyol et de cylcométhicone | |
| (Q2-3225C de la société DOW CORNING) | 20 % |
| Phényltriméthicone (Dow Corning 556 Fluid) | 4 % |
| Huile végétale | 3 % |
| Fibres de coton | 1 % |

| *Phase B* | |
|---|---|
| Glycérine | 23 % |
| Propylène glycol | 6 % |
| Hydroxyde de sodium | 1,8 % |
| Acide citrique | 1,2 % |
| Acide ascorbique | 5 % |
| Eau | qsp 100 % |

L'émulsion est préparée par préparation séparée des phases A et B et introduction de la phase B dans la phase A sous agitation.

On obtient une crème appropriée pour le soin du visage, douce à l'application, qui procure un éclate du teint immédiat et un estompage et lissage des imperfections de la peau.

### Exemple 6 : Emulsion H/E

| *Phase huileuse* | |
|---|---|
| Alcool stéarylique | 1 % |
| Arlacel 165 | 2 % |
| Cyclohexadiméthylsiloxane | 10 % |

| *Phase aqueuse* | |
|---|---|
| Vitamine B3 (niacinamide) | 2 % |
| Glycérine | 5 % |
| Carbomer | 0,2 % |
| Gomme de xanthane | 0,2 % |
| Hydroxyde de sodium | 0,01 % |
| EDTA (séquestrant) | 0,05 % |
| Conservateurs | 0,2 % |
| Aluminium Starch octenylsuccinate | |
| (Dry Flo de la société NATIONAL STARCH) | 3 % |
| Eau | qsp 100 % |
| Fibres de polyamide (Polyamide 0.9dtex, 0,3mm - société Paul Bonte) | 8 % |

L'émulsion est préparée par préparation séparée des phases A et B et introduction de la phase B dans la phase A sous agitation, puis addition des fibres à une température d'environ 40°C sous agitation.

On obtient une crème apte à dissimuler et traiter les imperfections de la peau ainsi qu'à rendre régulières la texture de la peau et/ou la taille des pores de la peau.

## Revendications

1. Utilisation cosmétique d'une composition cosmétique contenant des fibres et au moins un actif anti-vieillissement, pour camoufler les imperfections de la peau et traiter les signes du vieillissement de la peau.

2. Utilisation de fibres et d'au moins un actif anti-vieillissement, pour la préparation d'une composition pour application topique destinée au traitement du vieillissement de la peau tout en camouflant les signes du vieillissement.

3. Utilisation selon l'une quelconque des revendications précédentes, **caractérisée en ce que** les fibres ont une longueur (L) allant de 1 µm à 10 mm.

4. Utilisation selon l'une quelconque des revendications précédentes, **caractérisée en ce que** les fibres ont une section comprise dans un cercle de diamètre (D) allant de 1 nm à 100 µm.

5. Utilisation selon l'une quelconque des revendications précédentes, **caractérisée en ce que** les fibres ont un facteur de forme (L/D) allant de 5 à 150.

6. Utilisation selon l'une quelconque des revendications précédentes, **caractérisée en ce que** les fibres ont un titre allant de 0,15 à 30 deniers.

7. Utilisation selon l'une quelconque des revendications précédentes, **caractérisée en ce que** les fibres sont choisies parmi les fibres de soie, de coton, de laine, de lin, de cellulose extraites notamment du bois, des légumes ou des algues, de polyamide (Nylon®), de cellulose modifiée, de poly-p-phénylène téréphtalamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges.

8. Utilisation selon l'une quelconque des revendications précédentes, **caractérisée en ce que** les fibres sont enrobées et/ou fonctionnalisées.

9. Utilisation selon l'une quelconque des revendications précédentes, **caractérisée en ce que** les fibres sont choisies parmi les fibres de polyamide, les fibres de poly-p-phénylène téréphtalamide, les fibres de coton et leurs mélanges.

10. Utilisation selon l'une quelconque des revendications précédentes, **caractérisée en ce que** les fibres sont présentes en une quantité allant de 0,01 à 50 % en poids et de préférence de 1 à 20 % en poids par rapport au poids total de la composition.

11. Utilisation selon l'une quelconque des revendications précédentes, **caractérisée en ce que** l'actif anti-vieillissement est choisi parmi les agents hydratants, les agents anti-radicaux libres, les agents kératolytiques, les vitamines, les agents anti-élastase et anti-collagénase, les protides, les dérivés d'acides gras, les stéroïdes, les oligo-éléments, les agents blanchissants, les extraits d'algues et de planctons, les filtres solaires, les enzymes et co-enzymes, les flavonoïdes, les céramides, et leurs mélanges.

12. Utilisation selon l'une quelconque des revendications précédentes, **caractérisée en ce que** l'actif anti-vieillissement est un agent hydratant choisi parmi le lactate de sodium, les polyols, le mannitol, les acides aminés, l'acide hyaluronique; la lanoline; l'urée et les mélanges contenant de l'urée ; la vaseline ; et leurs mélanges.

13. Utilisation selon l'une quelconque des revendications précédentes, **caractérisée en ce que** l'actif anti-vieillissement est un agent anti-radicaux libres choisi parmi les dérivés de l'acide phosphonique, l'acide éthylènediamine tétracétique et ses sels, la guanosine, la superoxydismutase, le tocophérol et ses dérivés, l'éthoxyquine, la lactoferrine, la lactoperoxydase, et les dérivés nitroxydes, les superoxyde dismutases, le glutathion peroxydase, les extraits végétaux à activité antiradicalaire, et leurs mélanges.

14. Utilisation selon l'une quelconque des revendications précédentes, **caractérisée en ce que** l'actif anti-vieillissement est un agent kératolytique choisi parmi les α-hydroxy-acides notamment les acides dérivés de fruits, comme les acides glycolique, lactique, malique, citrique, tartrique, mandélique et leurs dérivés ; les β-hydroxy-acides comme l'acide salicylique et ses dérivés tels que l'acide n-octanoyl-5-salicylique ou l'acide n-dodécanoyl-5 salicylique ; les α-céto-acides comme l'acide ascorbique ou vitamine C et ses dérivés ; les β-céto-acides; les rétinoïdes comme le rétinol et ses esters, le rétinal, l'acide rétinoïque et ses dérivés et leurs mélanges.

15. Utilisation selon l'une quelconque des revendications précédentes, **caractérisée en ce que** l'actif anti-vieillissement est une vitamine choisie parmi la vitamine A, la vitamine C, la vitamine E, la vitamine B3, la vitamine B5, la vitamine D, la vitamine F, et leurs dérivés, analogues et précurseurs, et leurs mélanges.

16. Utilisation selon l'une quelconque des revendications précédentes, **caractérisée en ce que** l'actif anti-vieillissement est un agent anti-élastase ou un agent anti-collagénase choisi parmi les dérivés peptidiques, les inhibiteurs de métalloprotéase, la cystéine, les dérivés de N-acylamino-amides, et leurs mélanges.

17. Utilisation selon l'une quelconque des revendications précédentes, **caractérisée en ce que** l'actif anti-vieillissement est un protide choisi parmi les protéines, leurs hydrolysats, et leurs mélanges.

18. Utilisation selon l'une quelconque des revendications précédentes, **caractérisée en ce que** l'actif anti-vieillissement est un dérivé d'acide gras choisi parmi les phospholipides polyinsaturés et leurs mélanges.

19. Utilisation selon l'une quelconque des revendications précédentes, **caractérisée en ce que** l'actif anti-vieillissement est un stéroïde choisie parmi la DHEA, ses précurseurs biologiques, ses métabolites, et leurs mélanges.

20. Utilisation selon l'une quelconque des revendications précédentes, **caractérisée en ce que** l'actif anti-vieillissement est un oligo-élément choisi parmi le cuivre, le zinc, le sélénium, le fer, le magnésium, le manganèse et leurs mélanges.

21. Utilisation selon l'une quelconque des revendications précédentes, **caractérisée en ce que** l'actif anti-vieillissement est un agent blanchissant choisi parmi l'acide kojique et ses dérivés ; l'hydroquinone et ses dérivés ; l'arbutine et ses esters ; l'acide ellagique et ses dérivés ; les extraits de réglisse, de mûrier ou de scutellaire ; le glutathion et ses précurseurs ; la cystéine et ses précurseurs ; le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylaminocarbonyl-4-aminophénol ; et leurs mélanges.

22. Utilisation selon l'une quelconque des revendications précédentes, **caractérisée en ce que** l'actif anti-vieillissement est un extrait d'algue de la famille des Laminaires.

23. Utilisation selon l'une quelconque des revendications précédentes, **caractérisée en ce que** l'actif anti-vieillissement est un plancton en dispersion aqueuse.

24. Utilisation selon l'une quelconque des revendications précédentes, **caractérisée en ce que** l'actif anti-vieillissement est un filtre solaire choisi parmi les filtres chimiques UVA ou UVB, les filtres physiques et leurs mélanges.

25. Utilisation selon l'une quelconque des revendications précédentes, **caractérisée en ce que** l'actif anti-vieillissement est une enzyme choisie parmi les lipases, les protéases, les phospholipases, les cellulases, les peroxydases, les catalases, les superoxyde dimutases, les extraits végétaux contenant ces enzymes, et leurs mélanges.

26. Utilisation selon l'une quelconque des revendications précédentes, **caractérisée en ce que** l'actif anti-vieillissement est un co-enzyme choisi parmi le co-enzyme Q10, le co-enzyme R et leurs mélanges.

27. Utilisation selon l'une quelconque des revendications précédentes, **caractérisée en ce que** l'actif anti-vieillissement est une isoflavonoîde choisi parmi les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les α-méthyldeoxybenzoines, les 2-arylbenzofuranes, et leurs mélanges.

28. Utilisation selon l'une quelconque des revendications précédentes, **caractérisée en ce que** l'actif anti-vieillissement est un céramide choisi parmi la N-oléoyl-dihydrosphingosine, la N-stéaroyl-phytosphingosine, le N-α-hydroxybéhénoyl-dihydrosphingosine, la N-α-hydroxypalmitoyl-dihydrosphingosine, la N-linoléoyldihydrosphingosine, la N-palmitoyldihydrosphingosine, la N-stéaroyldihydrosphingosine, la N-béhénoyldihydrosphingosine, les glycocéramides, et leurs mélanges.

29. Utilisation selon l'une quelconque des revendications précédentes, **caractérisée en ce que** la quantité d'actif(s) anti-vieillissement(s) va de 0,0001 à 30 % en poids et de préférence de 0,01 à 20 % en poids de matière active par rapport au poids total de la composition.

30. Utilisation selon l'une quelconque des revendications précédentes, **caractérisée en ce que** la composition comprend un milieu physiologiquement acceptable comprenant de l'eau.

31. Utilisation selon l'une quelconque des revendications précédentes, **caractérisée en ce que** la composition est sous forme d'une émulsion.

32. Procédé de traitement cosmétique des signes du vieillissement d'une matière kératinique, **caractérisé en ce qu'**il consiste à appliquer sur la matière kératinique, une composition contenant des fibres et au moins un actif anti-vieillissement.

33. Procédé selon la revendication précédente, **caractérisé en ce que** la matière kératinique est la peau.

34. Composition pour application topique, contenant des fibres et au moins une vitamine choisie parmi la vitamine C, la vitamine B3, la vitamine B5, la vitamine D, la vitamine F, leurs dérivés, leurs précurseurs, leurs analogues, et leurs mélanges.

35. Composition selon la revendication 34, **caractérisée en ce que** les fibres sont conformes aux fibres selon l'une quelconque des revendications 3 à 9.

36. Composition selon la revendication 34 ou 35, **caractérisée en ce que** les fibres sont présentes en une quantité allant de 0,01 à 50 % en poids et de préférence de 1 à 20 % en poids par rapport au poids total de la composition.

37. Composition selon l'une quelconque des revendications 34 à 36, **caractérisée en ce que** la vitamine est choisie parmi l'acide ascorbique, ses sels, ses esters et ses sucres ; la niacinamide, l'acide nicotinique, l'alcool nicotinyle, l'acide nicotinurique, l'acide niconityl hydroxamique, et leurs dérivés ; le D-panthénol, le DL-panthénol, leurs dérivés et analogues, la gelée royale; la 1α,25-dihydroxyvitamine D3 et ses analogues ; les analogues de la vitamine D ; les mélanges d'acides insaturés possédant au moins une double liaison et les huiles végétales en contenant ; et leurs mélanges.

38. Composition selon l'une quelconque des revendications 34 à 37, **caractérisée en ce que** la quantité de vitamine(s) va de 0,0001 à 30 % en poids et de préférence de 0,01 à 20 % en poids de matière active par rapport au poids total de la composition.

39. Composition selon l'une quelconque des revendications 34 à 38, **caractérisée en ce qu'**elle comprend un milieu physiologiquement acceptable comprenant de l'eau.

40. Composition selon l'une quelconque des revendications 34 à 39, **caractérisée en ce qu'**elle est sous forme d'une émulsion.

41. Composition selon l'une quelconque des revendications 34 à 40, **caractérisée en ce qu'**elle contient en outre au moins un actif choisi parmi les hydratants, les actifs anti-radicaux libres, les α-hydroxy-acides, les β-hydroxy-acides, les rétinoïdes, les agents anti-élastase, les protides, les dérivés d'acide gras, les stéroïdes, les oligo-éléments, les agents blanchissants, les extraits d'algues, les planctons, les filtres solaires, les enzymes, les co-enzymes, les flavonoïdes, les céramides, et leurs mélanges.

42. Composition selon l'une quelconque des revendications 34 à 41, **caractérisée en ce qu'**elle constitue une composition cosmétique ou dermatologique.

43. Utilisation cosmétique d'une composition cosmétique selon l'une quelconque des revendications 34 à 41, pour un traitement cosmétique de la peau en vue de la tonifier, de la régénérer, de lisser les ridules de la peau, d'éclaircir le teint, d'atténuer les taches pigmentaires de la peau, et/ou pour lutter contre les méfaits des rayonnements U.V. et/ou pour enlever l'aspect huileux de la peau et/ou rendre réguliers la texture de la peau et/ou la taille des pores de la peau.

44. Procédé de traitement cosmétique des signes du vieillissement d'une matière kératinique, **caractérisé en ce qu'**il consiste à appliquer sur la matière kératinique, une composition cosmétique selon l'une quelconque des revendications 34 à 41.

45. Procédé selon la revendication précédente, **caractérisé en ce que** la matière kératinique est la peau.
